# EUROPEAN PATENT APPLICATION

(11) **EP 4 279 102 A2**
(43) Date of publication of application: **22.11.2023**
(21) Application number: 23199230.6
(22) Date of filing: 20.02.2020
(51) Int. Cl.: A61M 5/20

(54) **PEN NEEDLE**

(30) Priority: 12.03.2019 KR 20190028387; 19.12.2019 KR 20190170805
(62) Divisional of application: 20770666.4
(71) Applicant: Medexel Co., Ltd., Anseong-si, Gyeonggi-do 17535 (KR)
(72) Inventor: KIM, Hee Nam, 17163 Yongin-si Gyeonggi-do (KR)
(74) Representative: Richards, Michèle E.

(57) **Abstract**

An embodiment is a pen needle (10) which can be coupled to a pen-type syringe body. The pen needle (10) comprises: a needle (200); a needle hub (100) which comprises a coupling structure, which can be screw-coupled to a body, holds the needle (200) and has protrusions (110) formed on a supporting surface; a spring (300) which has one side supported on the supporting surface and provides resilience; a plunger (400) which has a plurality of resilient blades (W), which can be folded or unfolded, and has a hollow through which the needle (200) passes; a small cap (500) which is supported on the other side of the spring (300) and receives the plurality of blades (W) such that the plurality of blades can be received therein or be dislodged; and a medium cap (600) which is coupled to the small cap (500), accommodates the small cap (500) such that the small cap can move, and has serrations corresponding to the protrusions. When the upper surface of the small cap (500) is pressed, the plunger (400) moves together with the small cap (500) into the medium cap (600). When the upper surface of the small cap (500) is pressed and then the pressure is removed, the small cap (500) protrudes out of the medium cap (600) by means of the spring (300), and the plurality of blades (W) dislodged out of the small cap (500) are unfolded such that the small cap (500) does not move back into the medium cap (600). Due to the coupling of the protrusions (110) and serrations (610), the body and pen needle do not move independently from each other when coupled together.

## Description

### [Technical Field]

The technology relates to a pen needle.

### [Background Art]

A body of a pen type syringe accommodates medicine injectable from several times to dozens of times. The pen type syringe is configured so that a certain amount of medicine is injected into a human body by a pressing force of a user and used while a pen needle is replaced for each instance of use due to a risk such as infection and the like.

Since the pen needle is a single-use consumable but is repetitively reused or replaced by a user. In order to prevent a secondary infection caused by skin contact or stinging due to carelessness, pen needles including several functions to overcome these have been developed.

### [Technical Problem]

A pen needle and a body accommodating medicine according to the related art are screw-coupled to each other. When a user performs coupling by rotating the pen needle and/or body, a needle hub is not fixed and thus spins while being rotated in order to couple the pen needle to the syringe body. Accordingly, the user can not see whether the pen needle and the body are completely coupled, and thus a situation occurs in which injection into a human body is performed using the pen needle that is not completely coupled to the body.

The pen needle is a tool for simply injecting medicine into a patient such as a diabetic patient who needs regular injection. However, there were cases in which when a person such as the old or the infirm who had a low physical adjustability performed injection into his or her body, an injection needle did not perpendicularly penetrate his or her skin's surface, and thus medicine was not adequately transferred under the skin.

Also, a needle and a needle hub configured to grip the needle were included and the needle and the needle hub were coupled using an adhesive. During a process of manufacturing the needle hub, there were cases in which too little or too much of the adhesive was injected. When too little of the adhesive is injected, although a small force is applied, the needle may be separated from the needle hub. On the other hand, when too much of the adhesive is injected, a cured adhesive may flow out on a surface coming into contact with the user's skin while the pen needle is used such that a bad impression may be given to the user.

The present invention is directed to compensating for the above disadvantages of the pen needle according to the related art. A pen needle according to the embodiment may prevent a case in which medicine is not adequately transferred under the skin even when the old or the infirm performs injection, may prevent a separation of a needle caused by a small amount of adhesive, and may prevent leakage of adhesive caused by a large amount of adhesive.

The present invention is also directed to solving a problem of the related art by preventing a needle hub from spinning when a body configured to accommodate medicine is coupled to a pen needle.

### [Technical Solution]

One aspect of the present invention provides a pen needle to be coupled to a pen type syringe body. The pen needle includes a needle, a needle hub including a coupling structure screw-coupled to the body, configured to grip the needle, and having a support surface on which a protrusion is formed, a spring having one side supported by the support surface to provide an elastic force, a plunger including a plurality of wings having elasticity to be unfolded or folded and having a hollow through which the needle passes, a small cap supported by the other side of the spring and configured to accommodate the plurality of wings therein so that the plurality of wings are detached, and a medium cap coupled to the small cap, configured to accommodate the small cap so that the small cap moves, and on which a serration corresponding to the protrusion is formed. Here, when a top surface of the small cap is pressed, the plunger moves with the small cap into the medium cap, or when a pressure is removed after the top surface of the small cap is pressed, the small cap protrudes outward from the medium cap due to the spring and the plurality of wings dislodged from the small cap are unfolded so as not to allow the small cap to move into the medium cap again. Also, due to coupling between the protrusion and the serration, the body and the pen needle do not mutually spin while being coupled.

The spring may be accommodated by the small cap and the medium cap.

The needle hub and the medium cap may be coupled using coupling structures which are complementary to each other.

The complementary coupling structure formed on the needle hub may be a protrusion, and the complementary coupling structure formed on the medium cap may be a groove.

The complementary coupling structure formed on the needle hub may be a groove, and the complementary coupling structure formed on the medium cap may be a protrusion.

Another aspect of the present invention provides a pen needle to be coupled to a pen type syringe body. The pen needle includes a needle and a needle hub configured to grip the needle. Here, the needle hub includes a coupling structure coupled to the pen type syringe body, a post configured to grip the needle so that a near end portion of the needle is exposed in the coupling structure and a distal end portion of the needle is exposed to a surface of the needle hub, a needle hole in which a body of the needle is accommodated, and an adhesive storage portion configured to store an adhesive used to allow the needle and the needle hub to adhere to each other in the post. Here, a plurality of ribs are radially formed around the post and configured to increase a contact area with user's skin while injection is performed.

A leaking adhesive storage portion having a surface area increased in comparison to a surface area of the needle hole is formed on one end of the needle hole.

The leaking adhesive storage portion may have a shape in which any one of a cone, a cylinder, and a hexahedron may be engraved.

The pen needle may further include a cap configured to accommodate the needle and the needle hub, and a plane of the cap which accommodates the needle and the needle hub may be sterilized.

The cap may be press fitted to the needle hub.

The coupling structure may include a screw-coupling member screw-coupled to the pen type syringe and any one of a protrusion formed on one of the pen type syringe and the needle hub and a recess member formed on the other of the pen type syringe and the needle hub.

Still another aspect of the present invention provides a pen needle to be coupled to a pen type syringe body. The pen needle includes a needle and a needle hub including a coupling structure coupled to the pen type syringe body and configured to grip the needle. Here, the needle hub includes a post extending in a direction in which the needle extends and configured to grip the needle to allow a distal end portion of the needle to be exposed to a surface of the needle hub, a needle hole in which a body of the needle is inserted, an adhesive storage portion configured to store an adhesive used to allow the needle and the needle hub to adhere to each other in the post, and a leaking adhesive storage portion formed on one end of the needle hole and having a surface area increased in comparison to a surface area of the needle hole.

The pen needle may include a plurality of ribs radially formed around a center of the post and configured to increase an area in contact with user's skin while injection is performed.

The leaking adhesive storage portion may have a shape in which any one of a cone, a cylinder, and a hexahedron is engraved.

The pen needle may include a cap configured to accommodate the needle and the needle hub, and a plane of the cap which accommodates the needle and the needle hub may be sterilized.

The cap may be press fitted to the needle hub.

The coupling structure may include a screw-coupling member screw-coupled to the pen type syringe and any one of a protrusion formed on one of the pen type syringe and the needle hub and a recess member formed on the other of the pen type syringe and the needle hub.

### [Advantageous Effects]

According to the embodiment, since a pen needle and a body do not spin while being coupled, there is provided an advantage that a user can recognize whether the pen needle and the body are completely coupled.

In the pen needle according to the embodiment, there is provided an advantage that a post configured to accommodate a body of a needle includes an adhesive storage portion therein so as to prevent a separation of the needle caused by a lack of adhesive. According to one embodiment, a leaking adhesive storage portion configured to store a leaking adhesive is included so as to prevent a user from feeling uncomfortable due to adhesive leakage. There is provided an advantage that an area coming into contact with the user's skin is increased by a plurality of ribs so as to prevent an injection failure.

### [Description of Drawings]

FIG. 1 is an exploded perspective view of a pen needle 10 according to an embodiment.
FIG. 2A is a perspective view of a medium cap according to the embodiment, and FIG. 2B is a perspective view of a needle hub according to the embodiment.
FIG. 3 is a view illustrating a state in which a body and the pen needle are coupled.
FIGS. 4A to 4D are views illustrating a state of the pen needle according to the embodiment in use.
FIG. 5 is an exploded view of the pen needle according to the embodiment.
FIG. 6 is a cross-sectional view of a needle hub.
FIG. 7 is an enlarged cross-sectional view of a part of the needle hub.
FIG. 8A is a view illustrating the needle hub coupled to a needle, FIG. 8B is a view illustrating a state in which injection is performed using a pen needle according to the related art, and FIG. 8C is a view illustrating a state in which injection is performed using the pen needle according to the embodiment.
FIG. 9 is a cross-sectional view illustrating a state in which the needle hub configured to grip the needle is accommodated in a cap.

### [Modes of the Invention]

Since the following description of the present invention merely corresponds to an embodiment for describing a structure and functions of the present invention, the scope of the present invention should not be construed as being restricted by the embodiment described below. That is, since a variety of changes and a variety of shapes of the embodiment may be made, it should be understood that the scope of the present invention includes equivalents capable of implementing the technical concept thereof.

Meanwhile, the meaning of the terms described herein should be understood as follows.

The term "first," "second," or the like is intended to distinguish one component from another component, and the scope of the present invention should not be limited to the terms. For example, a first component may be designated as a second component, and similarly, the second component may be designated as the first component.

When it is stated that one component is "above" or "on" another component, it should be understood that the one component may be directly on the other component but another component may be present therebetween. On the other hand, when it is stated that one component is "in contact with" another component, it should be understood that no other component is present therebetween. Meanwhile, other expressions for describing a relationship between components, that is, "intervening in" and "directly intervening in," "between" and "directly between," "adjacent to" and "directly adjacent to," and the like should also be similarly construed.

It should be understood that singular expressions, unless clearly defined otherwise in context, include plural expressions. The terms "comprise," "have," and the like are used herein to specify the presence of stated features, numbers, stages, operations, elements, components or combinations thereof but do not preclude the presence or addition of one or more other features, numbers, stages, operations, elements, components, or combinations thereof.

Respective stages, unless a particular order is not definitely described in context, may be performed in an order different from a specified order. That is, the stages may be performed equal to the specified order, be performed substantially at the same time, or be performed in a reverse order.

Throughout the drawings referred to in order to describe the embodiments of the present disclosure, for convenience of description and ease of understanding, sizes, heights, thicknesses, and the like are intentionally exaggerated and may not be enlarged or reduced in accordance with a scale. In addition, in the drawings, any elements may be intentionally reduced and other elements may be intentionally enlarged.

All the terms used herein, unless defined otherwise, have the same meanings generally understood by one of ordinary skill in the art. Generally, terms defined in generally used dictionaries should be construed as having the meanings which coincide with contextual meanings in the art and cannot be construed as having ideal or excessively formal meanings unless clearly defined herein.

### First Embodiment

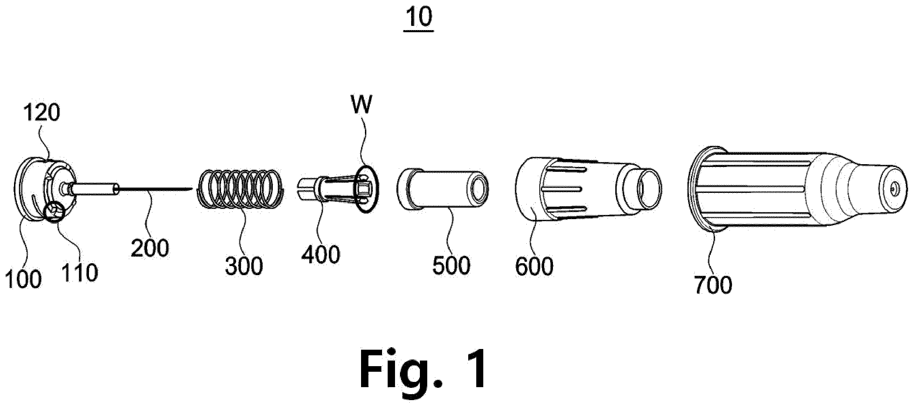

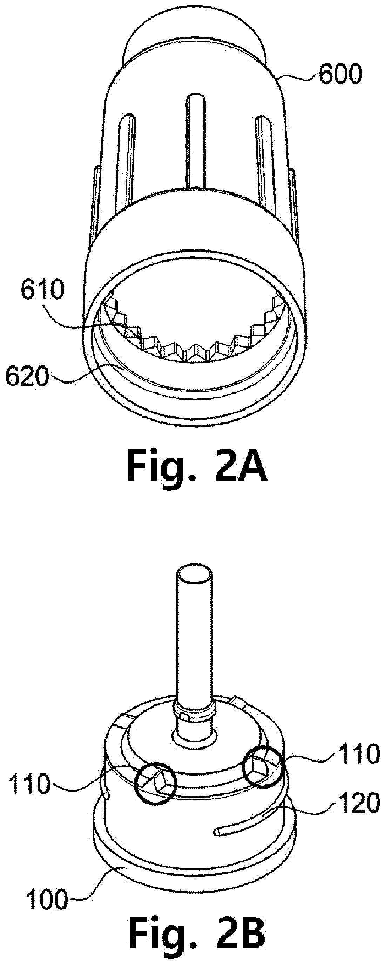

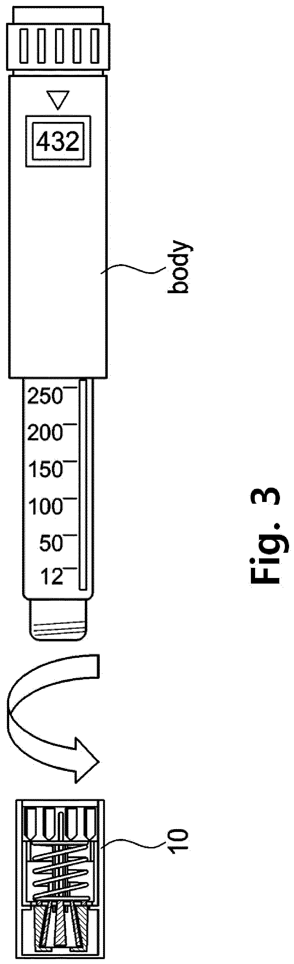

Hereinafter, a first embodiment will be described with reference to the attached drawings. FIG. 1 is an exploded perspective view of a pen needle 10 according to an embodiment. FIG. 2A is a perspective view of a medium cap 600 according to the embodiment, and FIG. 2B is a perspective view of a needle hub 100 according to the embodiment. Referring to FIGS. 1 to 2B, the pen needle 10 according to the embodiment includes a needle 200, the needle hub 100 including a coupling structure screw-coupled to a body (refer to FIG. 3), configured to grip the needle 200, and having one surface on which a first coupling member 110 is formed, a spring 300 having one side supported by the one surface to provide an elastic force, a plunger 400 including a plurality of wings W having elasticity to be folded or unfolded and a hollow through which the needle 200 passes, a small cap 500 supported by the other side of the spring 300 and in which the plurality of wings W are detachably accommodated, and a medium cap 600 coupled to the small cap 500, configured to accommodate the small cap 500 to allow the small cap 500 to be movable, and on which a serration 610 corresponding to a protrusion 110 is formed. When a top surface of the small cap 500 is pressed, the plunger 400 moves with the small cap 500 into the medium cap 600. When a pressure is removed after the top surface of the small cap 500 is pressed, the small cap 500 protrudes outward from the medium cap 600 due to the spring 300, and the plurality of wings 600 dislodged from the small cap 500 are unfolded so that the small cap 500 does not move again into the medium cap 600 so as not to mutually spin due to coupling between the first coupling member 110 and a second coupling member 610 when the body and the pen needle 10 are coupled.

The needle hub 100 grips the needle 200 and supports the spring 300 with one surface. The first coupling member 110 is formed on the one surface of the needle hub 100, and the first coupling member 110 is seated on the second coupling member 610 formed on the medium cap 600 as described below.

The needle hub 100 includes a coupling structure (not shown) configured to be screw-coupled to a syringe body. As an embodiment, the needle hub 100 and the syringe body may include screw structures which are formed to be complementary to each other, and for example, a female screw and a male screw may be formed on the needle hub 100 and the syringe body, respectively. As another example, a male screw and a female screw may be formed on the needle hub 100 and the syringe body, respectively.

The needle 200 also extends toward the syringe body and penetrates a human body to transfer medicine accommodated in the syringe body when the pen needle 10 and the body are coupled.

The plunger 400 includes a hollow, through which the needle 200 passes, and the plurality of wings W. The plunger 400 is formed of an elastic material, and the plurality of wings W are accommodated in the small cap 500 before the pen needle 10 is used. The small cap 500 is movably accommodated in the medium cap 600, and the small cap 500 comes into contact with the other side of the spring 300 so as to provide an elastic force.

The medium cap 600 is coupled to the small cap 500 and accommodates the small cap 500 so that the small cap 500 is movable. The medium cap 600 and the needle hub 100 are coupled to a complementary coupling structure 120 and 620 not to be disassembled. As shown in FIGS. 2A and 2B, the complementary coupling structure may be a protrusion 120 formed on the needle hub 100, and the protrusion 120 may be coupled to a groove 620 formed in the medium cap 600. In another embodiment which is not shown, the complementary coupling structure may be a protrusion formed on the medium cap 600, and the protrusion may be coupled to a groove formed in the needle hub 100.

FIG. 3 is a view illustrating a state in which the body and the pen needle 10 are coupled. According to the related art, although the body and/or the pen needle 10 should rotate to be coupled when the body and the pen needle 10 are coupled, there is no member supporting the needle hub 100, and thus the body and the needle hub 100 may mutually spin.

However, according to the embodiment shown in FIGS. 2A and 2B, when the body and the needle hub 100 are coupled, the first coupling member 110 formed on the needle hub 100 is seated on the second coupling member 610 formed in the medium cap 600. Accordingly, the needle hub 100 may be fixed to the medium cap 600, and it may be seen whether the body and/or pen needle 10 are completely coupled when rotated and coupled to each other.

As an example, when the body and/or the pen needle 10 are rotated and coupled to each other, the case in which the body and/or the pen needle 10 rotate without any resistance may be a case in which the body and the pen needle are not completely coupled. On the other hand, when the body and/or pen needle 10 are rotated and coupled to each other while jamming a plurality of times, the jamming may occur when the first coupling member and the second coupling member are dislodged from seated positions and seated in other positions due to a rotating force provided by a user. Here, the user may see that the body and the pen needle 10 are fully coupled.

In the embodiment shown in FIGS. 2A and 2B, the first coupling member 110 may be a protrusion and the second coupling member 610 may be a serration including both protrusions and grooves. In another embodiment which is not shown, the first coupling member may be a serration including both protrusions and grooves, and the second coupling member 610 may be a protrusion. In still another embodiment which is not shown, the first coupling member and the second coupling member may all be serrations.

In one embodiment, the pen needle 10 may further include a large cap 700 configured to accommodate the coupled needle hub 100 and medium cap 600. As one embodiment, the large cap 700 may include the needle hub 100 and the medium cap 600 which are coupled to a sterilized interior and may be sealed with sterilized paper after being accommodated.

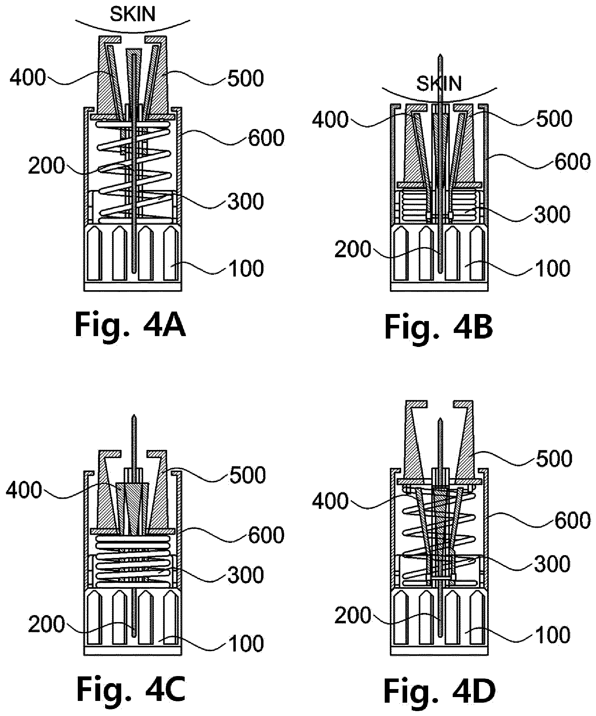

FIGS. 4A to 4D are views illustrating a state of the pen needle 10 according to the embodiment in use. Referring to FIG. 4A, the user may separate the pen needle 10 from the large cap 700 and brings the pen needle close to skin SKIN where injection is to be performed. The wings W of the plunger 400 are accommodated in the small cap 500.

FIG. 4B illustrates a state in which the user performs injection with the pen needle 10. Referring to FIG. 4B, when the user applies a pressure to the pen needle 10 to inject medicine, the small cap 500 in contact with the skin SKIN is pressed and moves into the medium cap 600 against an elastic force provided by the spring. The plunger 400 accommodated in the small cap 500 moves into the medium cap 600 as the small cap 500 moves. As the user applies the pressure to the pen needle 10, the needle 200 penetrates the skin SKIN where injection is to be performed.

FIG. 4C is a view illustrating the pen needle 10 spaced apart from the skin after injection is completed. Referring to FIG. 4C, according to the pen needle 10 being spaced apart from the skin, the spring 300 having one side supported by the needle hub 100 provides an elastic force to the small cap 500 connected to the other side and causes the small cap 500 to protrude outward from the medium cap 600. As the spring 300 provides the elastic force, the small cap 500 moves outward from the medium cap 600 while the wings of the plunger 400 are folded.

FIG. 4D is a view illustrating the pen needle 10 in which the wings W of the plunger 400 protrude outward from the small cap 500. Referring to FIG. 4D, the small cap 500 protrudes outward from the medium cap 600 due to the elastic force provided by the spring 300, and the plunger 400 is dislodged outward from the small cap 500 such that the wings W are unfolded again by the elastic force of the plunger 400.

Accordingly, even when an end of the small cap 500 is pressed, the small cap 400 does not move into the medium cap 600 due to the wings of the plunger 400 so that the small cap 500 is fixed. Also, as the small cap 500 is fixed, an end of the needle 200 is accommodated in the small cap 500 so that the pen needle 10 is prevented from being reused.

As one embodiment, the inside of the larger cap 700 may be sterilized, accommodate the pen needle 10, and be sealed with a sticker outside the large cap 700.

### Second Embodiment

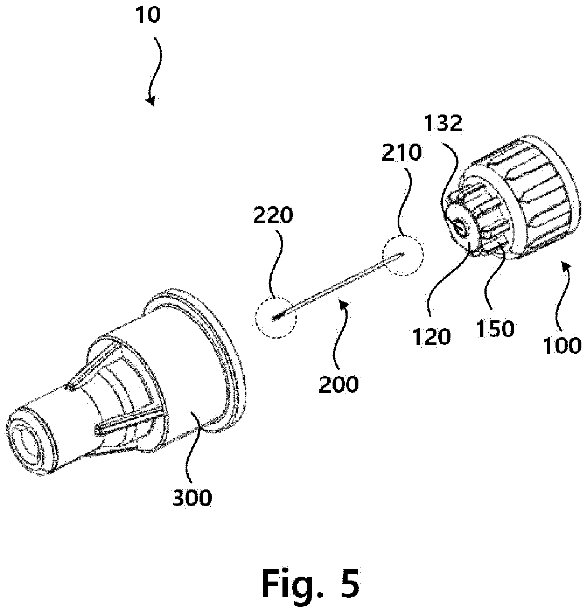

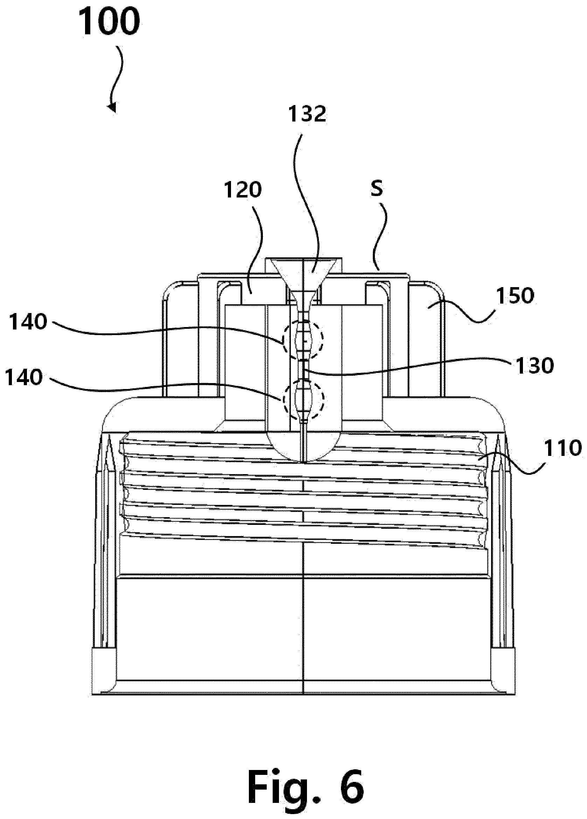

Hereinafter, an example of a general pen needle according to an embodiment will be described with reference to the attached drawings. FIG. 5 is an exploded view of the pen needle 1 according to the embodiment. FIG. 6 is a cross-sectional view of a needle hub 1000. Referring to FIGS. 5 and 6, the pen needle according to the embodiment is coupled to a pen type syringe body. The pen needle includes a needle 2000 and the needle hub 1000 configured to grip the needle 2000. The needle hub 1000 includes a coupling structure 1100 coupled to the pen type syringe body, a post 1200 configured to grip the needle so that a near end portion 2100 of the needle is exposed in the coupling structure 1100 and a distal end portion 2200 of the needle is exposed to a surface of the needle hub 1000, a needle hole 1300 in which a body of the needle 2000 is accommodated, an adhesive storage portion 1320 configured to store an adhesive, which allows the needle 2000 and the needle hub 1100 to adhere to each other, in the post, and a plurality of ribs 1500 radially formed around the post 1200 and configured to increase a contact area with the user's skin when injection is performed.

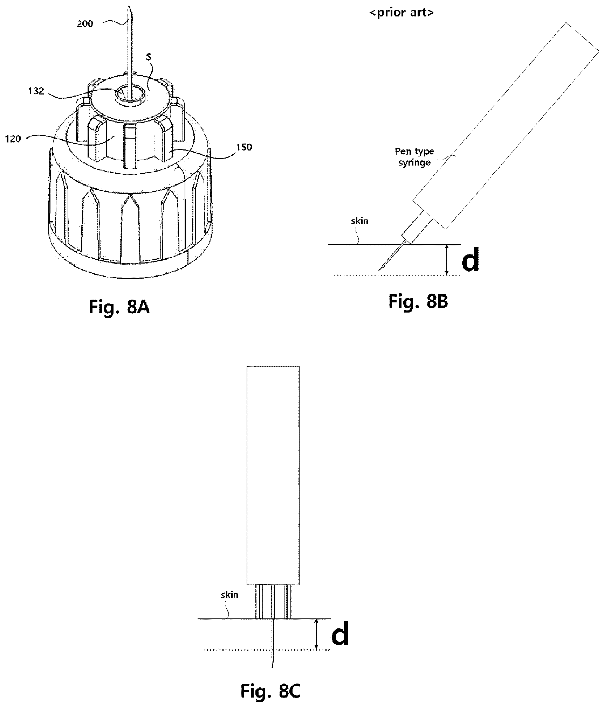

The needle 2000 is inserted into the needle hole 1300 formed in the needle hub 1000. The distal end portion 2200 of the needle 2000 is exposed from the post 1200 of the needle hub. An exposed length of the needle 2000 from the post 1200 is formed to be a length which allows the needle 2000 to penetrate the user's skin at a destination depth d (refer to FIG. 8) and to deliver medicine. As an example, the exposed length of the needle 2000 from the post 1200 may be 3.2 mm to 9.6 mm.

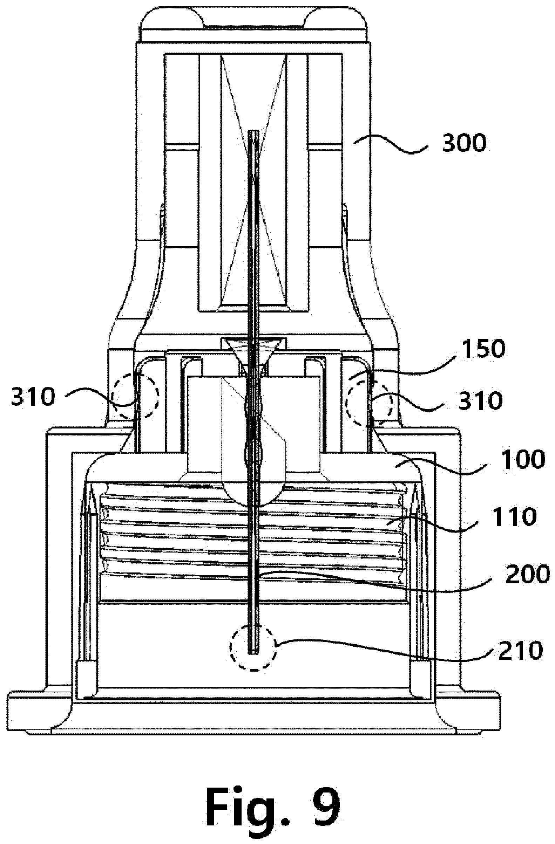

The near end portion 2100 of the needle extends into the coupling structure 1100 in which the pen needle 100 according to the embodiment is coupled to the pen type syringe body (not shown) and is exposed (refer to FIG. 9 for 200). The near end portion 2100 of the needle has a pointed end in order to penetrate the pen type syringe body (not shown) in which injection medicine is stored.

The needle hub 1000 includes the coupling structure 1100 to be coupled to the pen type syringe body (not shown). As shown in the illustrated embodiment, the coupling structure 1100 may be a screw member configured to be screw-coupled to the pen type syringe body (not shown), and a screw member corresponding thereto may be formed on a pen type syringe (not shown).

As another embodiment which is not shown, a coupling structure formed on a needle hub may be one of a protrusion coupled to a pen type syringe body and a recess member corresponding to the protrusion and the other of the protrusion and the recess member may be formed on the pen type syringe body.

The post 1200 extends in a direction in which the needle extends and accommodates the body of the needle 2000. The post 1200 includes the needle hole 1300 in which the needle is inserted. Through the needle hole 1300, the needle 2000 passes through the needle hub 2000, the near end portion 2100 of the needle is exposed to an inside of the coupling structure, and the distal end portion 2200 of the needle 2000 is exposed from a surface of the post 1200 to perform injection.

The adhesive storage portion 1400 is formed in the needle hole 1300. The needle 2000 is coupled to the needle hub 1000 using an adhesive. When too little of the adhesive is applied in a process of coupling the needle 2000 to the needle hub 1000, the needle 2000 may be separated from the needle hub 1000. When the needle 2000 is separated from the needle hub 1000 while medicine is being injected under the skin, a medical accident may occur.

In the embodiment, the adhesive storage portion 1400 configured to adequately store the adhesive is formed in the needle hub 1000. The adhesive storage portion 1400 includes a space capable of storing the adhesive along the needle hole 1300. When the needle 2000 is inserted through the needle hole 1300, the adhesive storage portion 1400 may provide an adequate amount of adhesive to a contact surface between the needle 2000 and the needle hole 1300 so as to firmly couple the needle 2000 to the needle hole 1300 and not be separated. Accordingly, the needle 2000 may be prevented from being separated from the needle hub 1000.

The adhesive storage portion 1400 may be formed to have a rounded shape to accommodate the adhesive therein like the illustrated example. As an example which is not shown, an adhesive storage portion may be formed to have an uneven shape which is angled, such as a perpendicular, triangle, and the like. Although FIG. 6 illustrates two adhesive storage portions 1400 formed along the needle hole 1300 as an example, one or two or more adhesive storage portions 1400 may be formed along the needle hole 1300.

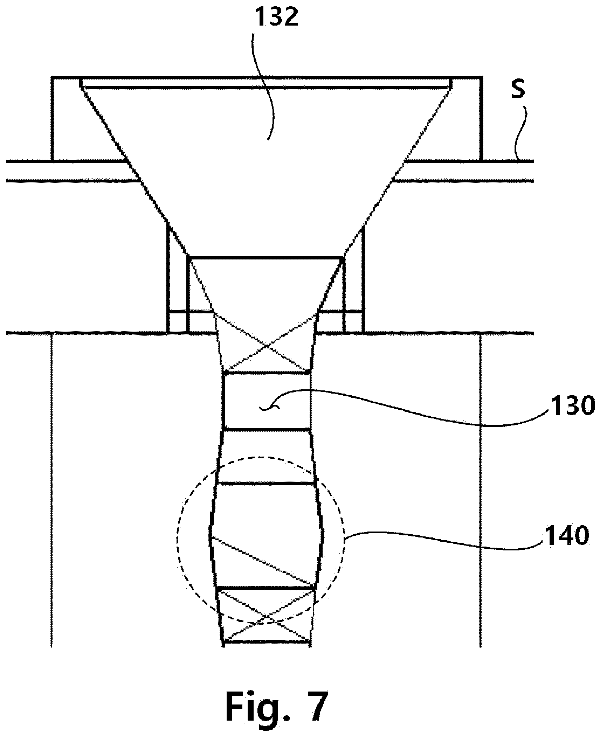

FIG. 7 is an enlarged cross-sectional view of a part of the needle hole 1300. Referring to FIG. 7, a leaking adhesive storage portion 1320 is formed in the needle hole 1300 extending upward from a top surface S of the post 1200. As described above, the needle 2000 and the needle hub 1000 are coupled to each other using the adhesive. When an adhesive is excessively injected from a pen needle according to the related art, the adhesive may flow outward from the top surface of the post 1200 and be cured while the pen needle is assembled by inserting the needle 2000 into the needle hole 1300. The adhesive which has flowed outward from the top surface of the post 1200 and has been cured may offend the user and give a perception of a defective product.

However, according to the embodiment, even when the excessively injected adhesive flows outward from the needle hole 1300, the adhesive is stored in the leaking adhesive storage portion 1320 and does not flow outward from the top surface S of the post. Accordingly, there is provided an advantage of providing furtherimproved feeling of use to the user.

Although the leaking adhesive storage portion 1320 is exemplified to have a conic shape with intaglio in the embodiment shown in FIG. 7, this depends on an embodiment and the leaking adhesive storage portion 1320 may have any shapes having a volume configured to store a leaking adhesive from the needle hole 1300. As an example, the leaking adhesive storage portion 1320 may have a hexahedral shape with intaglio. As another example, the leaking adhesive storage portion 1320 may have a cylindrical shape having a diameter greater than a diameter of the needle hole 1300. The adhesive stored in the leaking adhesive storage portion 1320 does not leak outward from the top surface S0 of the post 1200 and is stored in the leaking adhesive storage portion 1320 and cured.

FIG. 8A is a view illustrating the needle hub 1000 coupled to the needle 2000, FIG. 8B is a view illustrating a state in which injection is performed using a pen needle according to the related art, and FIG. 8C is a view illustrating a state in which injection is performed using the pen needle according to the embodiment. Referring to FIG. 8A, the needle hub 1000 according to the embodiment includes the plurality of ribs 1500 radially formed on the post 1200.

FIG. 8B is a view illustrating a state in which the pen needle according to the related art performs injection. Referring to FIG. 8B, the pen needle according to the related art has a small area in which a top surface of the pen needle comes into contact with the user's skin. Accordingly, as shown in the drawing, an angle at which the needle penetrates the user's skin does not form a 90-degree angle and diagonal penetration occurs. Accordingly, there is a disadvantage in which medicine is injected while the needle cannot penetrate deeper than the desired depth d under the skin skin such that efficiency in medicine delivery is reduced.

However, according to the embodiment shown in FIG. 8C, the plurality of ribs 1500 allow the needle 2000 to penetrate the skin perpendicularly when injection is performed using the pen needle. As an example, the ribs 1500 are radially formed around the needle hole 1300 formed in a center of the post 1200. Since the user may allow the needle 2000 to penetrate so that top surfaces of all the ribs 1500 come into contact with the user's skin skin and the ribs 1500 are radially formed around the post, the user may perform injection while seeing whether the ribs 1500 come into contact with the skin skin and may allow the needle 2000 to perpendicularly penetrate to a designated depth so as to deliver medicine. As shown in the drawing, eight ribs 1500 may be formed on the basis of the needle hole 1300. As another example, the number of ribs 1500 may be more than eight or less than eight.

In a conventional pen needle, a diameter of a post configured to grip the needle 2000 is smaller than or equal to one third of a diameter of a needle hub. That is, when the diameter of the needle hub is 12 mm in the conventional pen needle, the diameter of the post is 4 mm or less. Accordingly, in the pen needle according to the related art, as shown in FIG. 8C, since an area in contact with the user's skin is small, there are many cases of diagonally penetrating the user's skin.

However, a diameter of the post including the ribs in the pen needle according to the embodiment may be greater than or equal to half of a diameter of the needle hub, and more preferably, be greater than or equal to two-thirds thereof. Accordingly, the area in contact with the user's skin may be improved so as to allow the needle to perpendicularly penetrate under the user's skin.

FIG. 9 is a cross-sectional view illustrating a state in which the needle hub 1000 configured to grip the needle 2000 is accommodated in a cap 3000. Referring to FIG. 9, the cap 3000 accommodates the needle hub 1000 configured to grip the needle 2000 in an internal space. The internal space of the cap 3000 may be sterilized. An inside of the cap may be sealed with a sealing sticker (not shown) while accommodating the needle hub 1000 therein.

A protrusion member 3100 may be formed inside the cap 3000 to correspond to a sidewall of the rib 1500. The protrusion member 3100 of the cap 3000 and the sidewall of the rib 1500 rub each other so as to press fit the cap 3000 to the needle hub 1000. As an example, the protrusion member 3100 may be formed to have an annular shape at a position corresponding to the sidewall of the rib 1500 inside the cap 3000.

Although the embodiments shown in the drawings have been described above for reference in order to assist understanding the present invention, these are merely examples for implementation. It should be understood by one of ordinary skill in the art that a variety of modifications and equivalents thereof may be made therefrom. Accordingly, the technical scope of the present invention should be determined by the following claims.

### [Industrial Applicability]

Industrial applicability has been described above.

In addition to the earlier statements of invention, the invention provides:
1. A pen needle to be coupled to a pen type syringe body, the pen needle comprising:
   a needle;
   a needle hub including a coupling structure screw-coupled to the body, configured to grip the needle, and having a support surface on which a first coupling member is formed;
   a spring having one side supported by the support surface and configured to provide an elastic force;
   a plunger including a plurality of wings having elasticity to be unfolded or folded and having a hollow through which the needle passes;
   a small cap supported by the other side of the spring and configured to detachably accommodate the plurality of wings therein; and
   a medium cap coupled to the small cap, configured to movably accommodate the small cap, and on which a second coupling member corresponding to the first coupling member is formed,
   wherein when a top surface of the small cap is pressed, the plunger moves with the small cap into the medium cap, or when a pressure is removed after the top surface of the small cap is pressed, the small cap protrudes outward from the medium cap due to the spring, and the plurality of wings dislodged from the small cap are unfolded so as not to allow the small cap to move into the medium cap again, and
   wherein due to coupling between the first coupling member and the second coupling member, the body and the pen needle do not mutually spin while being coupled.
2. The pen needle of paragraph 1, wherein the first coupling member formed on the needle hub is a protrusion, and
   the second coupling member formed on the medium cap is a serration.
3. The pen needle of paragraph 1, wherein the first coupling member formed on the needle hub is a serration, and
   the second coupling member formed on the medium cap is a protrusion.
4. The pen needle of paragraph 1, wherein the spring is accommodated by the small cap and the medium cap.
5. The pen needle of paragraph 1, wherein the pen needle further comprises a large cap, and the pen needle is accommodated in the large cap which has been sterilized.
6. The pen needle of paragraph 1, wherein the needle hub and the medium cap are coupled using coupling structures which are complementary to each other.
7. A pen needle to be coupled to a pen type syringe body, the pen needle comprising:
   a needle; and
   a needle hub configured to grip the needle,
   wherein the needle hub comprises:
      a coupling structure coupled to the pen type syringe body;
      a post configured to grip the needle so that a near end portion of the needle is exposed in the coupling structure and a distal end portion of the needle is exposed to a surface of the needle hub;
      a needle hole in which a body of the needle is accommodated; and
      an adhesive storage portion configured to store an adhesive used to allow the needle and the needle hub to adhere to each other in the post, and
      a plurality of ribs are radially formed around the post and configured to increase a contact area with user's skin while injection is performed.
8. The pen needle of paragraph 7, wherein a leaking adhesive storage portion having a surface area increased in comparison to a surface area of the needle hole is formed at one end of the needle hole.
9. The pen needle of paragraph 8, wherein any one of a cone, a cylinder, and a hexahedron is engraved in the leaking adhesive storage portion.
10. The pen needle of paragraph 7, further comprising a cap configured to accommodate the needle and the needle hub,
   wherein a plane of the cap which accommodates the needle and the needle hub is sterilized.
11. The pen needle of paragraph 10, wherein the cap is press fitted to the needle hub.
12. The pen needle of paragraph 7, wherein the coupling structure comprises:
   a screw-coupling member screw-coupled to the pen type syringe; and
   any one of a protrusion formed on one of the pen type syringe and the needle hub and a recess member formed on the other of the pen type syringe and the needle hub.
13. A pen needle to be coupled to a pen type syringe body, the pen needle comprising:
   a needle; and
   a needle hub including a coupling structure coupled to the pen type syringe body and configured to grip the needle,
   wherein the needle hub comprises:
      a post extending in a direction in which the needle extends and configured to grip the needle to allow a distal end portion of the needle to be exposed to a surface of the needle hub;
      a needle hole in which a body of the needle is inserted;
      an adhesive storage portion configured to store an adhesive used to allow the needle and the needle hub to adhere to each other in the post; and
      a leaking adhesive storage portion formed on one end of the needle hole and having a surface area increased in comparison to a surface area of the needle hole.
14. The pen needle of paragraph 13, comprising a plurality of ribs radially formed around a center of the post and configured to increase an area in contact with user's skin while injection is performed.
15. The pen needle of paragraph 13, wherein any one of a cone, a cylinder, and a hexahedron is engraved in the leaking adhesive storage portion.
16. The pen needle of paragraph 13, comprising a cap configured to accommodate the needle and the needle hub, and
   wherein a plane of the cap which accommodates the needle and the needle hub is sterilized.
17. The pen needle of paragraph 16, wherein the cap is press fitted to the needle hub.
18. The pen needle of paragraph 13, wherein the coupling structure comprises:
   a screw-coupling member screw-coupled to the pen type syringe; and
   any one of a protrusion formed on one of the pen type syringe and the needle hub and a recess member formed on the other of the pen type syringe and the needle hub.

## Claims

1. A pen needle to be coupled to a pen type syringe body, the pen needle comprising:
a needle; and
a needle hub,
wherein the needle hub comprises:
a coupling structure adapted to be coupled to the pen type syringe body;
a post configured to accommodate the needle so that a proximal end portion of the needle extends into the coupling structure and a distal end portion of the needle is exposed from a surface of the needle hub/the post;
a needle hole provided in the needle hub/the post in which a body of the needle is accommodated; and
an adhesive storage portion configured to store an adhesive used to allow the needle and the needle hub to adhere to each other in the post, and
a plurality of ribs radially formed around the post and configured to increase a contact area with user's skin while injection is performed.

2. The pen needle of claim 1, wherein a leaking adhesive storage portion having a surface area increased in comparison to a surface area of the needle hole is formed at one end of the needle hole.

3. The pen needle of claim 2, wherein any one of a cone, a cylinder, and a hexahedron is engraved in the leaking adhesive storage portion.

4. The pen needle of claim 1, further comprising a cap configured to accommodate the needle and the needle hub,
wherein a plane of the cap which accommodates the needle and the needle hub is sterilized.

5. The pen needle of claim 4, wherein the cap is press fitted to the needle hub.

6. The pen needle of claim 1, wherein the coupling structure comprises:
a screw-coupling member adapted to be screw-coupled to the pen type syringe; and
any one of a protrusion formed on one of the pen type syringe and the needle
hub and a recess member formed on the other of the pen type syringe and the needle hub.

7. A pen needle to be coupled to a pen type syringe body, the pen needle comprising:
a needle; and
a needle hub including a coupling structure adapted to be coupled to the pen type syringe body and configured to accommodate the needle,
wherein the needle hub comprises:
a post extending in a direction in which the needle extends and configured to accommodate the needle to allow a distal end portion of the needle to be exposed from a surface of the needle hub;
a needle hole in which a body of the needle is inserted;
an adhesive storage portion configured to store an adhesive used to allow the needle and the needle hub to adhere to each other in the post; and
a leaking adhesive storage portion formed on one end of the needle hole and having a surface area increased in comparison to a surface area of the needle hole.

8. The pen needle of claim 7, comprising a plurality of ribs radially formed around a center of the post and configured to increase an area in contact with user's skin while injection is performed.

9. The pen needle of claim 7, wherein any one of a cone, a cylinder, and a hexahedron is engraved in the leaking adhesive storage portion.

10. The pen needle of claim 7, comprising a cap configured to accommodate the needle and the needle hub, and
wherein a plane of the cap which accommodates the needle and the needle hub is sterilized.

11. The pen needle of claim 10, wherein the cap is press fitted to the needle hub.

12. The pen needle of claim 7, wherein the coupling structure comprises:
a screw-coupling member adapted to be screw-coupled to the pen type syringe; and
any one of a protrusion formed on one of the pen type syringe and the needle hub and a recess member formed on the other of the pen type syringe and the needle hub.
